(19) Europäisches Patentamt
European Patent Office
Office européen des brevets

(11) Publication number: 0 025 912
B1

(12) # EUROPEAN PATENT SPECIFICATION

(45) Date of publication of patent specification: 30.07.86

(51) Int. Cl.$^4$: C 07 D 311/82, G 01 N 33/58, C 09 K 11/07

(21) Application number: 80105253.1

(22) Date of filing: 03.09.80

(54) **2,7-Dialkylfluorescein derivatives and their use in fluorescence immunoassays.**

(30) Priority: 07.09.79 US 73158

(43) Date of publication of application:
01.04.81 Bulletin 81/13

(45) Publication of the grant of the patent:
30.07.86 Bulletin 86/31

(84) Designated Contracting States:
DE FR GB IT SE

(56) References cited:
DE-A-2 422 337
DE-A-2 824 917
US-A-3 996 345
US-A-3 998 943
US-A-4 199 559

CRC, Atlas of Spectral Data and Physical constants for Organic Compounds, Grasselli, CRC Press p. B 993, Steroids, Fieser and Fieser, Reinhold N.Y. 1959, pp. 15-21

(73) Proprietor: SYVA COMPANY
3181 Porter Drive
Palo Alto, CA 94304 (US)

(72) Inventor: Khanna, Pyare Lal
5442 Kaveny Drive
San Jose, California (US)
Inventor: Ullman, Edwin Fisher
135 Selby Lane
Atherton, California (US)

(74) Representative: Vossius Vossius Tauchner Heunemann Rauh
Siebertstrasse 4 P.O. Box 86 07 67
D-8000 München 86 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 025 912 B1

**Description**

Fluorescent label compound in particular fluorescein, rhodamine and tetramethylrhodamine find use in fluorescent immunoassays; see US patents 3 996 345 and 3 998 943. Of particular interest is the use of fluorescent label compounds in the form of ligand conjugates and receptor conjugates to be used in the determination of a variety of ligands, both antigens and receptors. A substantial proportion of the ligands are assayed in physiological fluids, such as serum, where the serum can provide significant background fluorescence. One way to diminish the background fluorescence resulting from naturally present fluorescers is to use a fluorescent label compound which absorbs at relatively long wavelengths. The compound should desirably have a large Stokes shift, be stable under conditions of the assay, be relatively free of non-specific interference, both from materials in solution and the compound to which the fluorescent label compound is conjugated and to provide high quantum yields. In addition, for certain applications, it is desirable that the fluorescent label compound be coupled with a quencher molecule, that is a molecule which is capable of absorbing the energy of the fluorescent label compound in the excited state when within a predetermined distance, so that the fluorescent label compound does not fluoresce.

It is, therefore, an object of this invention to provide novel conjugates of 2,7-dialkyl-substituted-9-substituted xanthen-6-hydroxy-3H-3-ones (fluorescein derivatives) with members of specific binding pairs, ligands and receptors. The conjugates find a wide variety of uses, particularly as reagents in immunoassays as described e.g. in US patents 3 996 345 and 3 998 943. The fluorescein derivatives preferably have at least two chloro substituents at other than the 1,8-positions and have a linking group, either aliphatic or aromatic, in the 2- or 9-position of the xanthene nucleus for linking to a member of a specific binding pair. Particularly preferred is the 9-position for the linking group.

The fluorescent label compounds, i.e. the fluorescein derivatives contain in their molecule at least about 15 carbon atoms, generally 21 to about 40 carbon atoms, usually from about 22 to 36 carbon atoms. There will preferably be at least two chloro substituents at other than the 1,8-positions and the there may be as many as 6 chloro substituents. In addition to chlorine atoms, the only other hetero atoms in the molecule are bromine, chalcogen, particularly oxygen and sulfur, and nitrogen atoms. The fluorescein derivatives contain at least 4 hetero atoms and usually not more than 20 hetero atoms, more usually not more than about 16 hetero atoms and preferably not more than about 16 hetero atoms and preferably not more than about 12 hetero atoms. Of the hetero atoms other than chlorine atoms, there will be at least 3 oxygen more usually at least 5 oxygen atoms. Apart from the oxygen atoms which are part of the xanthene chromophore, there are oxygen atoms in the form of non-oxo-carbonyl oxygen atoms or ether oxygen atoms (oxy groups), particularly in the form of acid, ester or ether groups (normally bonded solely to carbon and hydrogen); sulfur is normally present in the form of sulfonyl, thioether or mercapto groups. Nitrogen is normally present in the form of amino or amido groups (bonded solely to carbon and hydrogen). The term "non-oxo-carbonyl" or "non-oxo-carbonyl" group basically covers any group in which a carbonyl group, i.e. a carbon atom linked to a carbonyl oxygen atom, is bound to a hetero atom, i.e. not a carbon or hydrogen atom. Thus, a simple example is a carboxyl group. It also includes such groups as ester, amido, acid anhydride and acyl halide groups.

The fluorescein derivatives are further characterized by having absorption maxima in 0.05 *M* phosphate buffer pH 8 of at least about 500 nm, an extinction coefficient in the same medium of at least about 65,000, more usually at least 70,000 and a Stokes shift in the same medium of at least about 10 nm, more usually at least about 12 nm.

The conjugates of the 9-substituted-2,7-dialkylsubstituted xanthenones of this invention may have the following general formula:

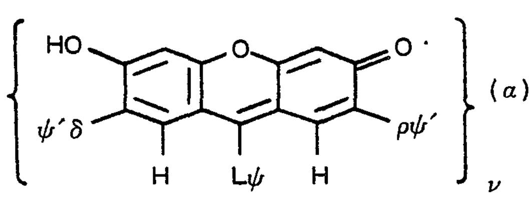

wherein:

p is an aliphatic group, normally an aliphatic hydrocarbon group saturated or unsaturated, branched or straight chain, particularly an alkylene group, more particularly $(CH_2)_\varepsilon$, wherein ε is of from 1 to 12, usually 1 to 6, and preferably 1 to 4; p contains normally 1 to 12, usually 1 to 6, and preferably 1 to 4 carbon atoms;

the two ψ's are the same or different, normally being the same, except when linking to α, and are hydrogen atoms, a non-oxo-carbonyl linking group or one of the ψ's may be a non-oxo-carbonyl linking group;

L is a bond or divalent group, usually an organic group containing 1 to 20 carbon atoms, usually not more than 16, and preferably not more than 10 carbon atoms, normally having an aliphatic or aromatic hydrocarbon chain, or combination thereof.

The aliphatic chain contains usually about 2 to 6 carbon atoms and the aromatic chain about 6 to 12, usually 6 to 10 carbon atoms; L normally has from 0 to 4, when aromatic, usually 1 to 4, more usually 2 to 4 substituents, wherein the substituents may be halogen atoms particularly chlorine atoms, non-oxo-carbonyl groups, thio groups, including inert sulfur acids, esters and amides, amino groups, particularly *tert*-amino groups or amido groups and oxy groups, wherein the substituents contain normally from 0 to 4 carbon atoms, there being at least two carbon atoms between hetero atoms bonded to saturated carbon atoms,

α is an organic compound, a member of a specific binding pair, either a ligand or a receptor; α is covalently bonded to one of ψ or ψ'. The covalent bond normally involves an amido, methylene sec-amino, ether, thioether or azo link;

ψ is a group terminating in a hetero atom containing functional group when not bonded to α, wherein the terminal hetero atom containing functional group may be bonded directly to a carbon atom of L or through an oligomer of from 1 to 4 units, each unit containing 1 to 4, usually 2 to 4 carbon atoms, which units are amino acids, alkyleneamino, or alkyleneoxy groups. The terminal functional group is normally an oxo group, including an oxo-carbonyl and a non-oxo-carbonyl group, an amino; oxy or thio group or an active halogen atom particularly a non-oxo-carbonyl group; and

μ is on the average at least one and not more than the molecular weight of α divided by 500, usually divided by 1,000.

Desirably, there are from 2 to 6 chloro substituents on the fluorescein derivative (in the brackets), bonded at other than the 1,8-positions of the xanthenone. Also, the 4,5-positions may be unsubstituted or one or both, usually both positions are substituted with bromine or chlorine atoms or with alkyl groups containing 1 to 6, usually 1 to 3 carbon atoms.

The conjugate with the organic compound (α) may be linked, covalently or non-covalently to a support. The conjugate may be bound either through the fluorescein derivative or organic compound. The support will be described in greater detail subsequently.

Furthermore the xanthenone conjugates of this invention may have the following general formula:

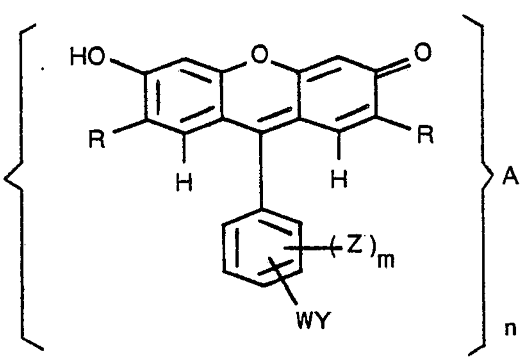

wherein:

R is an aliphatic group of from 1 to 8, usually 1 to 6, preferably 1 to 4, and especially 1 to 3 carbon atoms, which may be substituted or unsubstituted, aliphatically saturated or unsaturated. Particularly preferred is an alkyl or carboxyalkyl group containing 1 to 6, usually 1 to 4 carbon atoms;

Z is a carboxy group;

W is a bond or divalent group containing from 0 to 16, either 0 or usually 1 to 16 carbon atoms, preferably 1 to 8 carbon atoms and from 0 to 10, usually 2 to 8 hetero atoms. The hetero atoms are chalcogen (oxygen and sulfur) or nitrogen atoms. Chalcogen atoms are bonded solely to carbon atoms (oxy or oxo) and nitrogen atoms are bonded solely to carbon and hydrogen atoms (amino and amido). The carbon atoms are normally aromatic or aliphatic, particularly free of aliphatic unsaturation; W is conveniently a monomer or an oligomer containing per unit 1 to 4 carbon atoms, e.g. an alkylene, aminoacid, oxyalkylene, aminoalkylen group, etc.;

Y may be taken together with A to form an active functional group capable of forming a covalent bond with a heterofunctional group, such as an amino, hydroxy or mercapto group present on A, when A is not taken together with Y. When Y is taken together with A to give an active functional group, such active functional groups include oxo- and non-oxo-carbonyl, oxy, thio, amino, active halo, active olefin and inorganic acyl groups (e.g. sulfonyl, etc). When not taken together with A, Y acts as a linking group, containing either a methylene group or a heteroatom.

A, when not taken together with Y, is a member of a specific binding pair, which is a ligand or receptor, wherein the ligand may be haptenic or antigenic, normally having a molecular weight from about 125 to an indefinite upper limit. For the most part, most ligands will have a molecular weight under 10 million, more usually under 2 million, with varying ranges depending upon the nature of the ligand or receptor;

m will be 0 to 3, more usually 0 to 2; and

n will be 1 when Y and A are taken together.

Otherwise n will have a value of 1 to x, where x is the molecular weight of A divided by 500, more usually divided by 1,000, and more frequently divided by 2,000. When A has a molecular weight greater than 600,000, n will normally be not greater than A divided by 5,000. In addition, there will usually be at least two chlorine atoms bonded on any of the available positions where no specific atom is indicated.

Also, the 4,5-positions may be substituted as described previously. Furthermore, either the conjugate or the fluorescein derivative may be bonded to a support having a molecular weight of at least about 10,000 and up to an indefinite upper limit.

A preferred group of xanthenone conjugates has the following general formula:

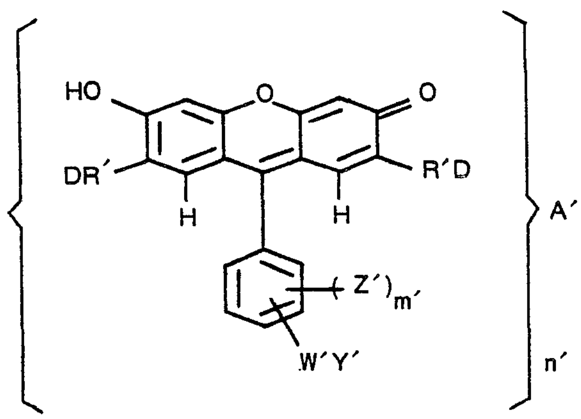

wherein:

R' is an alkylene group containing 1 to 6, usually 1 to 4, and preferably 1 to 3 carbon atoms;

D is a hydrogen atom or a carboxy group;

Z' is a carboxy group;

m' is 0 to 3, usually 0 to 2;

Y' may be taken together with A' to form an active functional group which may be a non-oxo-carbonyl group, including the sulfur analog thereof, an amino group bonded to at least one hydrogen atom, a mercapto group, an active ethylene group usually having an α-carbonyl group, a halogenmethylcarbonyl group, wherein the halogen is chlorine, bromine or iodine, a sulfonyl group, or the like; when not taken together with A', Y' will be a linking group, either a methylene group or a hetero atom containing linking group, usually an amide, ester, ether or azo linking group;

W' is a bond or linking group containing 1 to 16 usually 1 to 12, and preferably 1 to 8 atoms other than hydrogen atoms, which are carbon, nitrogen, oxygen or sulfur atoms, preferably carbon, nitrogen and oxygen atoms, there being from 0 to 8 carbon atoms and 0 to 8 hetero atoms, with the number of carbon atoms and hetero atoms being at least 1, wherein nitrogen atoms will be bonded solely to hydrogen and carbon atoms, and will be either amino or amido groups. Oxygen and sulfur atoms will be bonded solely to carbon atoms as oxy (thio) or oxo (thiono) groups. The carbon atoms are normally aliphatic and usually free of aliphatic unsaturation; W' may be alkylene or alkenylene group containing 1 to 8, usually 1 to 4 carbon atoms, an oxoalkylene or oxoalkenylene group containing 1 to 8, usually 1 to 4 carbon atoms, an imino (NH), N-formyl amino acid or N-formyl poly (amino acid) group e.g. a glycine or polyglycine group, there being from about 1 to 4 amino acid groups, with the terminal carboxy group being Y'A', or the like;

n' will be 1 when Y' and A' are taken together. Otherwise n' will have a value of 1 to x, where x is the molecular weight of A' divided by 500, more usually divided by 1,000 and more frequently divided by 2,000. When A' has a molecular weight greater than 500,000, n will normally be not greater than A' divided by 5,000; there generally being not more than 5 carboxyl groups, usually not more than 4 carboxyl groups in total, and there being from 0 to 6, preferably 2 to 5 chlorine atoms bonded to available carbon atoms; and

A' is a member of a specific binding pair, a ligand or receptor, wherein the ligand may be haptenic or antigenic.

The 4,5-positions are preferably unsubstituted or chloro-substituted.

In addition, the above conjugate may be bonded to a support.

For the most part, the fluorescein derivatives to be employed for conjugation will have the following general formula:

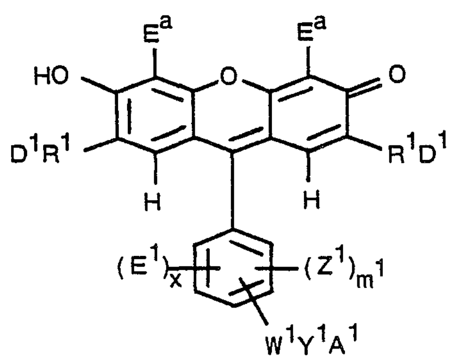

wherein:

$R^1$ is an alkylene group containing 1 to 6, usually 1 to 4, preferably 1 to 2 carbon atoms;

$D^1$ is a hydrogen atom or a carboxy group, preferably a hydrogen atom;

$Z^1$ is a carboxy group;

$E^a$ is a hydrogen atom, an alkyl group containing 1 to 6, usually 1 to 3 carbon atoms, or a chlorine atom;

$E^1$ is a chlorine atom

$W^1$ is a bond or linking group containing 1 to 12, usually 1 to 8 atoms other than hydrogen atoms and generally 1 to 8, usually 1 to 6 atoms in the chain wherein the atoms are carbon, nitrogen, oxygen and sulfur atoms, particularly carbon, nitrogen and oxygen atoms, wherein the carbon atoms are aliphatic. The nitrogen atoms are present as amido or amino groups, particularly as amino groups bonded solely to carbon atoms. Oxygen and sulfur atoms are bonded solely to carbon atoms and are oxy or oxo groups or the sulfur analogs thereof;

$W^1$ will generally be an aliphatic group, being saturated or unsaturated, normally saturated, having from 0 to 1 site of ethylenic unsaturation, an alkylene or alkenylene group containing 1 to 8, usually 1 to 4 carbon atoms, a N-formal amino acid or a N-formal poly(amino acid) group, where the terminal carboxy group is derived from $Y^1A^1$, an amino or mercapto group, or the like;

$Y^1A^1$ are taken together to form a linking group, wherein $Y^1A^1$ is bonded solely to carbon or nitrogen atoms, with the proviso that when $Y^1$ and $A^1$ are bonded to nitrogen, $Y^1A^1$ is carbonyl, including the nitrogen and sulfur analogs thereof and can be doubly bonded to nitrogen;

$Y^1A^1$ can be a non-oxo-carbonyl or a haloacetyl group, a halogen atom, particularly a chlorine or bromine atom, a maleimido, mercapto, amino, or inorganic acyl group having a phosphorous or a sulfur atom as the central atom;

$m^1$ is 0 to 3, usually 0 to 2, there usually being not more than a total of 5 carboxyl groups in the molecule, usually not more than a total of 4 carboxyl groups, and preferably not more than about 2 carboxyl groups, other than $Y^1A^1$;

x is 0 to 4, preferably 2 to 4, there generally being not more than a total of 6 chlorine atoms in the molecule, usually not more than a total of 4 chlorine atoms, wherein x plus $m^1$ is not greater than 4.

For the most part, the xanthenone conjugates of this invention when bonded to a ligand or support will have the following general formula:

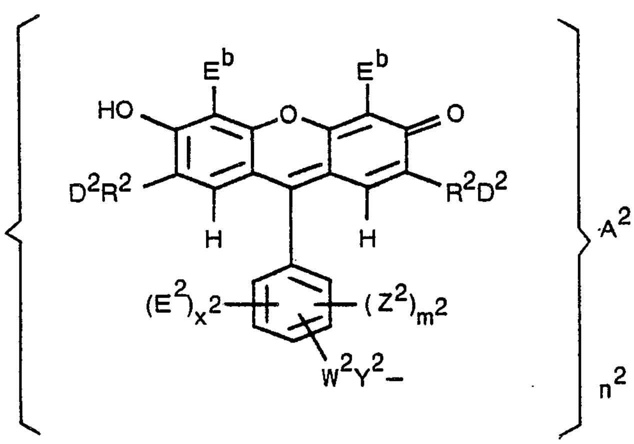

wherein:

$E^b$ is a hydrogen or chlorine atom;

$E^2$ is a chlorine atom;

$Z^2$ is a carboxy group;

$R^2$ is an alkylene group containing 1 to 6, usually 1 to 3, preferably 1 to 2 carbon atoms;

$D^2$ is a hydrogen atom or a carboxy group, preferably a hydrogen atom;

$W^2$ is a bond or linking chain. The linking chain contains 1 to 12, usually 1 to 10, and preferably 1 to 8 atoms other than hydrogen atoms, and about 1 to 10, usually 1 to 8, and preferably 1 to 6 atoms in the chain or spacer arm, wherein the atoms are carbon, oxygen, nitrogen and sulfur, particularly carbon, oxygen and nitrogen in the spacer arm. The oxygen and sulfur atoms are bonded solely to carbon atoms in the form of oxy or oxo groups. The nitrogen atoms are bonded solely to carbon and hydrogen atoms in the form of amino and amido groups. The hetero atoms bonded to saturated carbon atoms are separated by at least two carbon atoms;

$W^2$ is particularly an alkylene, carboxamidoalkylene or poly(carboxamidoalkylene) group of the formula $(-CONH(CH_2)_{1-2}-)_a$ wherein a is in the range of from about 1 to 4, usually 1 to 3.

$Y^2$ is a non-oxo-carbonyl, carbamoyl, thiocarbamoyl, sulfonyl, methylene, amino, oxy or thio group, particularly a functional group having a non-oxo-carbonyl group or sulfur analog thereof;

$x^2$ is 0 to 4;

$m^2$ is 0 to 3, preferably 1 to 2;

$n^2$ is 1 to x, where x is the molecular weight of $A^2$ divided by 500, usually divided by 1,000, and more usually divided by 2,000. When $A^2$ is a ligand having a molecular weight of about 125 to 2,000, $n^2$ will generally be of from about 1 to 20. When $A^2$ is a ligand having a molecular weight of from about 2,000 to 600,000, $n^2$ will generally be in the range of about 1 to 100, more usually in the range of about 2 to 50; and

$A^2$ is a ligand having a molecular weight of at least about 125 and up to 10 million or more, which is haptenic or antigenic. The haptens have a molecular weight from about 125 to 2,000. The antigens will generally have a molecular weight from about 5,000 to 10 million, more usually from about 5,000 to 2 million and frequently from about 5,000 to 600,000. The ligand is a member of a specific binding pair, which comprises a compound bearing at least one determinant or epitope site and a receptor which is capable of recognizing the determinant site. $A^2$ is a receptor having a molecular weight of from about 10,000 to 1 million.

The haptenic ligands will include compounds of antigenic, and haptenic ligands will include compounds of interest such as drugs, hormones, pollutants, compounds of interest in processing, agricultural chemicals, metabolites, and the like. Antigens will primarily be proteins, poly-saccharides or nucleic acids, individually or in combination with each other or other materials, such as occurring in cells, viruses, phages, or the like. The haptens will normally have a molecular weight from about 125 to 2,000, more usually to 1,000 while the antigens will normally have a molecular weight from about 2,000, more usually 5,000 up to an indefinite molecular weight, usually not exceeding 10 million, more usually not exceeding 2 million.

Finally, in some instances it may be desirable to have the fluorescent label compound or the conjugate of the fluorescent label compound with the ligand, bonded to a support, where the linkage may be derived from either the fluorescent label compound or the ligand, normally the ligand. In this situation, the linking group may be any convenient functional group which is present on the fluorescent label compound or the ligand or a functional group which may introduced, particularly on the ligand. These xanthenone conjugates have the following general formula, where the symbols are derived from the previous general formula for the conjugate for the most part:

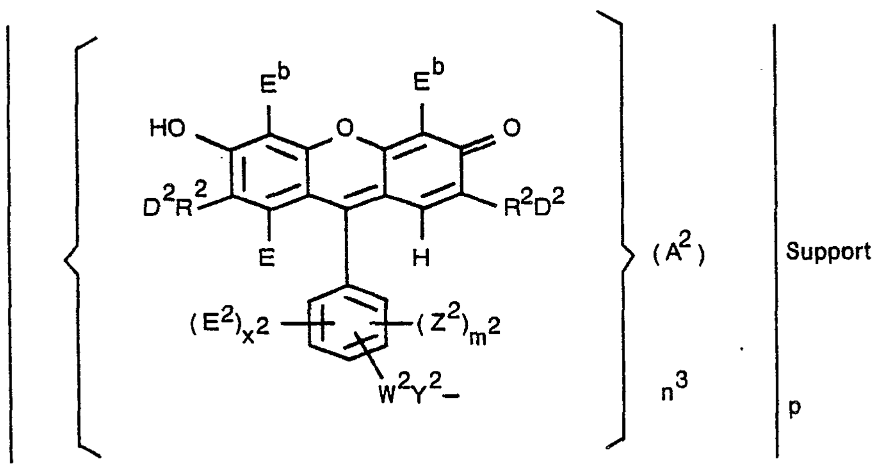

wherein:

all of the symbols have been defined previously, except for:

$n^3$ which is at least 1 and up to the molecular weight of $A^2$ divided by 500, usually 1000, more usually 1,500, with the proviso that when q is 0, $n^3$ is 1;

p which is at least 1 and of up to the molecular weight of the support divided by 500, more usually the molecular weight of the support divided by 1,000, wherein when the molecular weight of the support exceeds 500,000, p will normally be not greater than the molecular weight of the support divided by 5,000, more usually divided by 10,000.

Turning now to a consideration of the individual components of the xanthenone conjugates, the fluorescein derivatives will be considered first. Table I contains a list of illustrative fluorescein derivatives to be used.

TABLE I

2,7-dimethyl-4,5-dichloro-9-(2',4',5'-tricarboxyphenyl)-6-hydroxy-3-xanthen-3-one

2,7-diethyl-4,5-dichloro-9-(2',4',5'-tricarboxy-3',6'-dichlorophenyl)-6-hydroxy-3H-xanthen-3-one

2,7-dihexyl-9-(2',4',5'-tricarboxyphenyl)-6-hydroxy-3H-xanthen-3-one

2,7-dimethyl-4,5-dichloro-9-(2'-carboxy-4'-isothiocyanato-3',5'-dichlorophenyl)-6-hydroxy-3H-xanthen-3-one

2,7-dimethyl-9-(2'-carboxy-4'-isocyanato-3',5',6'-trichlorophenyl)-6-hydroxy-3H-xanthen-3-one

2,7-dimethyl-9-(4'-carboxy-5'-carboxylphenyl)glycylglycylglycine amide 6-hydroxy-3H-xanthen-3-one

2,7-di(carboxymethyl)-9-(4',5'-dicarboxy-2',3',6'-trichlorophenyl)-6-hydroxy-3H-xanthen-3-one

2,7-di(carboxypropyl)-4,5-dichloro-9-(3′,4′-dicarboxyphenyl)-6-hydroxy-3H-xanthen-3-one
2,7-diethyl-9-(2′-carboxy-4′-amino-3′,5′-dichlorophenyl)-6-hydroxy-3H-xanthen-3-one
2,7-dimethyl-9-(2′-carboxy-4′-mercaptophenyl)-6-hydroxy-3H-xanthen-3-one
2,7-dimethyl-9-(2′-carboxy-4′-carboxymethylphenyl)-6-hydroxy-3H-xanthen-3-one
2,7-dimethyl-9-(2′-carboxy-4′-(4″-carboxybutyl)phenyl)-6-hydroxy-3H-xanthen-3-one
2,7-dimethyl-4,5-dichloro-9-(2′,4′-dicarboxy-5′-(carboxamidomethylene)phenyl)-6-hydroxy-3H-xanthen-3-one
2,7-dimethyl-4,5-dichloro-9(3′-carboxypropyl)-6-hydroxy-3H-xanthen-3-one.

As indicated previously, the fluorescein derivatives will be conjugated with ligands and/or supports. The following is a description of the applicable ligands.

*Analyte*

The ligand analytes are characterized by being monoepitopic or polyepitopic. The polyepitopic ligand analytes will normally be poly(amino acids) i.e. polypeptides and proteins, polysaccharides, nucleic acids, and combinations thereof. Such combinations of assemblages include bacteria, viruses, chromosomes, genes, mitochondria, nuclei, cell membranes, and the like.

For the most part, the polyepitopic ligand analytes employed in the subject invention will have a molecular weight of at least about 5,000, more usually at least about 10,000. In the poly(amino acid) category, the poly(amino acids) of interest will generally have a molecular weight from about 5,000 to 5,000,000, more usually from about 20,000 to 1,000,000 and in particular from about 2,000 to 600,000; among the hormones of interest, the molecular weights will usually range from about 5,000 to 60,000.

The wide variety of proteins may be considered as to the family of proteins having similar structural features, proteins having particular biological functions, proteins related to specific microorganisms, particularly disease causing microorganisms, etc.

The following are classes of proteins related by structure:

protamines
histones
albumins
globulins
scleroproteins
phosphoproteins
mucoproteins
chromoproteins
lipoproteins
nucleoproteins
glycoproteins
proteoglycans
unclassified proteins, e.g. somatotropin, prolactin, insulin, pepsin

A number of proteins found in the human plasma are important clinically and include:

Prealbumin
Albumin
$\alpha_1$-Lipoprotein
$\alpha_1$-Acid glycoprotein
$\alpha_1$-Antitrypsin
$\alpha_1$-Glycoprotein
Transcortin
4.6S-Postalbumin
Tryptophan-poor
$\alpha_1$-glycoprotein
$\alpha_1$X-Glycoprotein
Thyroxin-binding globulin
Inter-$\alpha$-trypsin-inhibitor
Gc-globulin
(Gc 1—1)
(Gc 2—1)
(Gc 2—2)
Haptoglobin
(Hp 1—1)
(Hp 2—1)
(Hp 2—2)
Ceruloplasmin
Cholinesterase
$\alpha_2$-Lipoprotein(s)
Myoglobin
C-Reactive Protein

$\alpha_2$-Macroglobulin
$\alpha_2$-HS-glycoprotein
Zn-$\alpha_2$-glycoprotein
$\alpha_2$-Neuramino-glycoprotein
Erythropoietin
$\beta$-lipoprotein
Transferrin
Hemopexin
Fibrinogen
Plasminogen
$\beta_2$-Glycoprotein I
$\beta_2$-Glycoprotein II
Immunoglobulin G
(IgG) or $\gamma$G-globulin
Mol. formula:
$\gamma_2\kappa_2$ or $\gamma_2\lambda_2$
Immunoglobulin A (IgA)
or $\gamma$A-globulin
Mol. formula:
$(\alpha_2\kappa_2)^n$ or $(\alpha_2\lambda_2)^n$
Immunoglobulin M
(IgM) or $\gamma$M-globulin
Mol. formula:
$(\mu_2\kappa_2)^5$ or $(\mu_2\lambda_2)^5$
Immunoglobulin D(IgD)
or $\gamma$D-Globulin ($\gamma$D)
Mol. formula:
$(\delta_2\kappa_2)$ or $\delta_2\lambda_2)$
Immunoglobulin E (IgE)
or $\gamma$E-Globulin ($\gamma$E)
Mol. formula:
$(\varepsilon_2\kappa_2)$ or $(\varepsilon_2\lambda_2)$
Free $\kappa$ and $\lambda$ light chains
Complement factors:
C'1
C'1q
C'1r
C'1s
C'2
C'3
$\beta_1$A
$\alpha_2$D
C'4
C'5
C'6
C'7
C'8
C'9

Important blood clotting factors include:

BLOOD CLOTTING FACTORS

| International designation | Name |
|---|---|
| I | Fibrinogen |
| II | Prothrombin |
| IIa | Thrombin |
| III | Tissue thromboplastin |
| V and VI | Proaccelerin, accelerator globulin |
| VII | Proconvertin |
| VIII | Antihemophilic globulin (AHG) |
| IX | Christmas factor, plasma thromboplastin component (PTC) |
| X | Stuart-Prower factor, autoprothrombin III |
| XI | Plasma thromboplastin antecedent (PTA) |
| XII | Hagemann factor |
| XIII | Fibrin-stabilizing factor |

Important protein hormones include:

*Peptide and Protein Hormones*
Parathyroid hormone
(parathromone)
Thyrocalcitonin
Insulin
Glucagon
Relaxin
Erythropoietin
Melanotropin
(melanocyte-stimulating hormone; intermedin)
Somatotropin
(growth hormone)
Corticotropin
(adrenocorticotropic hormone)
Thyrotropin
Follicle-stimulating hormone
Luteinizing hormone
(interstitial cell-stimulating hormone)
Luteomammotropic hormone
(luteotropin, prolactin)
Gonadotropin
(chorionic gonadotropin)

*Tissue Hormones*
Secretin
Gastrin
Angiotensin I and II
Bradykinin
Human placental lactogen

*Peptide Hormones from the Neurohypophysis*
Oxytocin
Vasopressin
Releasing factors (RF)
CRF, LRF, TRF, Somatotropin-RF, GRF, FSH—RF, PIF, MIF

Other polymeric materials of interest are mucopolysaccharides and polysaccharides. Illustrative antigenic polysaccharides derived from microorganisms are as follows:

| *Species of Microorganisms* | *Hemosensitin Found in* |
|---|---|
| Streptococcus pyogenes | Polysaccharide |
| Diplococcus pneumoniae | Polysaccharide |
| Neisseria meningitidis | Polysaccharide |
| Neisseria gonorrheae | Polysaccharide |
| Corynebacterium diphtheriae | Polysaccharide |
| Actinobacillus mallei:<br>Actinobacillus whitemori | Crude extract |
| Francisella tularensis | Lipopolysaccharide<br>Polysaccharide |
| Pasteurella pestis | |
| Pasteurella pestis | Polysaccharide |
| Pasteurella multocida | Capsular antigen |
| Brucella abortus | Crude extract |
| Haemophilus influenzae | Polysaccharide |
| Haemophilus pertussis | Crude |
| Treponema reiteri | Polysaccharide |
| Veillonella | Lipopolysaccharide |
| Erysipelothrix | Polysaccharide |
| Listeria monocytogenes | Polysaccharide |
| Chromobacterium | Lipopolysaccharide |
| Mycobacterium tuberculosis | Saline extract of 90% phenol extracted mycobacteria and polysaccharide fraction of cells and tuberculin |
| Klebsiella aerogenes | Polysaccharide |
| Klebsiella cloacae | Polysaccharide |
| Salmonella typhosa | Lipopolysaccharide,<br>Polysaccharide |
| Salmonella typhi-murium:<br>Salmonella derby<br>Salmonella pullorum | Polysaccharide |
| Shigella dysenteriae | Polysaccharide |

| *Species of Microorganisms* | *Hemosensitin Found in* |
| --- | --- |
| Shigella flexneri | |
| Shigella sonnei | Crude, Polysaccharide |
| Rickettsiae | Crude extract |
| Candida albicans | Polysaccharide |
| Entamoeba histolytica | Crude extract |

The microorganisms which are assayed may be intact, lysed, ground or otherwise fragmented, and the resulting composition or portion, e.g. by extraction, assayed. Microorganisms of interest include:

*Corynebacteria*
Corynebacterium diptheriae

*Pneumococci*
Diplococcus pneumoniae

*Streptococci*
Streptococcus pyogenes
Streptococcus salivarus

*Staphylococci*
Staphylococcus aureus
Staphylococcus albus

*Neisseriae*
Neisseria meningitidis
Neisseria gonorrheae

*Enterobacteriaciae*

| | |
| --- | --- |
| Escherichia coli<br>Aerobacter aerogenes<br>Klebsiella pneumoniae | The coliform bacteria |
| Salmonella typhosa<br>Salmonella choleraesuis<br>Salmonella typhimurium | The Salmonellae |
| Shigella dysenteriae<br>Shigella schmitzii<br>Shigella arabinotarda<br>Shigella flexneri<br>Shigella boydii<br>Shigella sonnei | The Shigellae |

*Other enteric bacilli*

| | |
| --- | --- |
| Proteus vulgaris<br>Proteus mirabilis<br>Proteus morgani | Proteus species |

Pseudomonas aeruginosa
Alcaligenes faecalis
Vibrio cholerae

*Hemophilus-Bordetella group*
Hemophilus influenzae, H. ducreyi
H. hemophilus
H. aegypticus
H. parainfluenzae
Bordetalla pertussis

*Pasteurellae*
- Pasteurella pestis
- Pasteurella tulareusis

*Brucellae*
- Brucella melitensis
- Brucella abortus
- Brucella suis

*Aerobic Spore-forming Bacilli*
- Bacillus anthracis
- Bacillus subtilis
- Bacillus megaterium
- Bacillus cereus

*Anaerobic Spore-forming Bacilli*
- Clostridium botulinum
- Clostridium tetani
- Clostridium perfringens
- Clostridium novyi
- Clostridium septicum
- Clostridium histolyticum
- Clostridium tertium
- Clostridium bifermentans
- Clostridium sporogenes

*Mycobacteria*
- Mycobacterium tuberculosis hominis
- Mycobacterium bovis
- Mycobacterium avium
- Mycobacterium leprae
- Mycobacterium paratuberculosis

*Actinomycetes* (fungus-like bacteria)
- Actinomyces israelii
- Actinomyces bovis
- Actinomyces naeslundii
- Nocardia asteroides
- Nocardia brasiliensis

*The Spirochetes*
- Treponema pallidum
- Treponema pertenue
- Treponema carateum
- Borrelia recurrentis
- Leptospira icterohemorrhagiae
- Leptospira canicola
- Spirillum minus
- Streptobacillus moniliformis

*Mycoplasmas*
- Mycoplasma pneumoniae

*Other pathogens*
- Listeria monocytogenes
- Erysipelothrix rhusiopathiae
- Streptobacillus moniliformis
- Donvania granulomatis
- Bartonella bacilliformis

*Rickettsiae* (bacteria-like parasites)
- Rickettsia prowazekii
- Rickettsia mooseri
- Rickettsia rickettsii
- Rickettsia conori
- Rickettsia australis
- Rickettsia sibiricus
- Rickettsia akari
- Rickettsia tsutsugamushi
- Rickettsia burnetii
- Rickettsia quintana

*Chlamydia* (unclassifiable parasites bacterial/viral)
- Chlamydia agents (naming uncertain)

*Fungi*
- Cryptococcus neoformans
- Blastomyces dermatidis
- Histoplasma capsulatum
- Coccidioides immitis
- Paracoccidioides brasiliensis
- Candida albicans
- Aspergillus fumigatus
- Mucor corymbifer (Absidia corymbifera)

- Rhizopus oryzae
- Rhizopus arrhizus
- Rhizopus nigricans

} Phycomycetes

- Sporotrichum schenkii
- Fonsecaea pedrosoi
- Fonsecaea cómpacta
- Fonsecaea dermatidis
- Cladosporium carrionii
- Phialophora verrucosa
- Aspergillus nidulans
- Madurella mycetomi
- Madurella grisea
- Allescheria boydii
- Phialosphora jeanselmei
- Microsporum gypseum
- Trichophyton mentagrophytes
- Keratinomyces ajelloi
- Microsporum canis
- Trichophyton rubrum
- Microsporum andouini

*Viruses*

*Adenoviruses*

*Herpes Viruses*
- Herpes simplex
- Varicella (Chicken pox)
- Herpes Zoster (Shingles)
- Virus B
- Cytomegalovirus

*Pox Viruses*
- Variola (smallpox)
- Vaccinia
- Poxvirus bovis
- Paravaccinia
- Molluscum contagiosum

*Picornaviruses*
Poliovirus
Coxsackievirus
Echoviruses
Rhinoviruses

*Myxoviruses*
Influenza (A, B, and C)
Parainfluenza (1—4)
Mumps Virus
Newcastle Disease Virus
Measles Virus
Rinderpest Virus
Canine Distemper Virus
Respiratory Syncytial Virus
Rubella Virus

*Arboviruses*
Eastern Equine Encephalitis Virus
Western Equine Encephalitis Virus
Sindbis Virus
Chikugunya Virus
Semliki Forest Virus
Mayora Virus
St. Louis Encephalitis Virus
California Encephalitis Virus
Colorado Tick Fever Virus
Yellow Fever Virus
Dengue Virus

*Reoviruses*
Reovirus Types 1—3

*Hepatitis*
Hepatitis A Virus
Hepatitis B Virus

*Tumor Viruses*
Rauscher Leukemia Virus
Gross Virus
Maloney Leukemia Virus

The monoepitopic ligand analytes will generally have a molecular weight from about 100 to 2,000, more usually from 125 to 1,000. The analytes of interest include drugs, metabolites, pesticides, pollutants, and the like. Included among drugs of interest are the alkaloids. Among the alkaloids are morphine alkaloids, which includes morphine, codeine, heroin, dextromethorphan, their derivatives and metabolites; cocaine alkaloids, which includes cocaine and benzoyl ecgonine, their derivatives and metabolites; ergot alkaloids, which includes the diethylamide of lysergic acid; steroid alkaloids; iminazoyl alkaloids; quinazoline alkaloids, isoquinoline alkaloids; quinoline alkaloids; which includes quinine and quinidine; diterpene alkaloids; their derivatives and metabolites.

The next group of drugs includes steroids, which include the estrogens, gestagens, androgens, andrenocortical steroids, bile acids, cardiotonic glycosides and aglycones, which include digoxin and digoxigenin, saponins and sapogenins, their derivatives and metabolites. Also included are the steroid mimetic substances, such as diethylstilbestrol.

The next group of drugs includes lactams having from 5 to 6 annular members, which include the barbiturates, e.g. phenobarbital and secobarbital, diphenylhydantonin, primidone, ethosuximide, and their metabolites.

The next group of drugs includes aminoalkylbenzenes, with alkyl of from 2 to 3 carbon atoms, which include the amphetamines, catecholamines, which include ephedrine, L-dopa, epinephrine, narceine, papaverine, their metabolites and derivatives.

The next group of drugs includes benzheterocyclics which include oxazepam, chlorpromazine, tegretol, imipramine, their derivatives and metabolites, the heterocyclic rings being azepines, diazepines and phenothiazines.

The next group of drugs includes purines, which include theophylline, caffeine, their metabolites and derivatives.

The next group of drugs includes those derived from marijuana, which includes cannabinol and tetrahydrocannabinol.

The next group of drugs includes the vitamins such as A, B, e.g. $B_{12}$, C, D, E and K, folic acid, thiamine.

The next group of drugs includes prostaglandins, which differ by the degree and sites of hydroxylation and unsaturation.

The next group of drugs includes antibiotics, e.g. include penicillin, chloromycetin, actinomycetin, tetracycline, terramycin, their metabolites and derivatives, aminoglycosides, such as gentamicin, kanamicin, tobramycin, and amikacin.

The next group of drugs includes the nucleosides and nucleotides, e.g. ATP, NAD, FMN, adenosine, guanosine, thymidine, and cytidine with their appropriate sugar and phosphate substituents.

The next group of drugs comprises miscellaneous individual drugs which include methadone, meprobamate, serotonin, meperidine, amitriptyline, nortriptyline, lidocaine, procaineamide, acetylprocaineamide, propranolol, griseofulvin, valproic acid, butyrophenones, antihistamines, anticholinergic drugs, such as atropine, their metabolites and derivatives.

The next group of compounds comprises amino acids and small peptides which include polyiodothyronines e.g. thyroxine, and triiodothyronine, oxytocin, ACTH, angiotensin, met- and leu-enkephalin their metabolites and derivatives.

Metabolites related to disease states include spermine, galactose, phenylpyruvic acid, and porphyrin Type 1.

Among pesticides of interest are polyhalogenated biphenyls, phosphate esters, thiophosphates, carbamates, polyhalogenated sulfenamides, their metabolites and derivatives.

For receptor analytes, the molecular weights will generally range from 10,000 to $2 \times 10^6$, more usually from 10,000 to $10^6$. For immunoglobulins, IgA, IgG, IgE and IgM, the molecular weights will generally vary from about 160,000 to about $10^6$. For enzymes the molecular weight will normally range from about 10,000 to 6000,000. The molecular weights of natural receptors varies widely, generally from at least about 25,000 to $10^6$ or even higher. Examples of such materials are avidin, thyroxine binding globulin, thyroxine binding prealbumin, transcortin, etc.

In many applications it is desirable to have the ligand bonded to a support, either directly, through the intermediacy of a ligand, or directly to the support, while bound to a ligand.

A wide variety of supports may be employed.

Various supports may be employed, both soluble or insoluble, swellable or nonswellable, by aqueous or organic solvents, naturally occurring or synthetic, organic or inorganic, porous or nonporous, or the like. Various polymeric materials include vinyl polymers and copolymers, polysaccharides, silicones, glass, carbon particles, such as graphite or charcoal, metals or metal compounds, poly(amino acids), nucleic acids or the like.

The term support denotes a macromolecular support having a molecular weight of at least about 10,000, which may be naturally ~~occurring~~ or synthetic, having a plurality of linking groups e.g. carboxy, hydroxy, or amino groups, usually being a polymer, such as a polysaccharide or an addition polymer. The support is bonded to the conjugate by any convenient functional group remaining on $A^2$ or the conjugate in the brackets. The particular manner of linking is not a significant aspect of the present invention. For example, if $A^2$ is a poly(amino acid) group, carboxylic groups on the support can be used for amide formation or maleimide groups may be introduced and linked to mercapto groups.

The particles or supports can be derived from naturally occurring materials, naturally occurring materials which are synthetically modified and synthetic materials. Of particular interest are polysaccharides, particularly crosslinked polysaccharides, such as agarose, which is available as Sepharose®, dextran, available as Sephadex® and Sephacyl®, cellulose, starch, and the like. Other materials include polyacrylamides, polystyrene, polyvinyl alcohol, copolymers of hydroxyethyl methacrylate and methyl methacrylate, silicones, glasses, available as Bioglas®, nucleic acids, poly(amino acids), cells or the like. In addition to solid particles, liquid particles may also be employed having a lipophilic or amphiphilic membrane, which serves to contain an internal fluid and define a space. Such particles include vesicles, cells and liposomes.

The particles may be porous or nonporous, swellable or nonswellable by aqueous or organic media, normally having a variety of functional groups, such as hydroxyl, amino or carboxy groups, either bonded directly to the backbone or by means of a spacer arm, crosslinked or noncrosslinked, smooth or rough surface, or the like.

The porous particles may have a wide variety of molecular weight cut off sizes, generally varying from about 10,000 to many million usually not exceeding 20 million.

As already indicated, a wide variety of linking chains may be employed between the fluorescein derivative and the ligand and/or support. The choice of linking group will vary widely, depending upon the functional groups present in the fluorescein derivative or functional groups which may be readily introduced, the desired length of the linking group, the desirability of having the linking group provide for a particular environment, chemical property or physical property, e.g. positively or negatively charged, solubility enhancement, dipole effects, or the like.

Table II indicates a variety of linking groups which may be employed for linking the fluorescein derivatives to the ligand:

TABLE II

| Functional group of fluorescein derivative | Linking group | Functional group of ligand |
|---|---|---|
| —COOH | $—(NH—G—CO)_g—$ | $—NH_2$ |
| —COOH or $—SO_3H$ | $—(NH—G'—NH)_{g'}—NH—G'—NH—$ | —COOH |
| | $—NH—G'—N(CH_2CH_2)_2N—G'—NH—$ | |
| —SH | 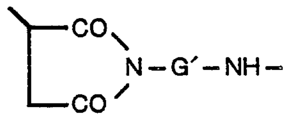 | —COOH |
| $—CO—CH_2$-halo | —O—G—NH— | —COOH |
| | —S—G—NH— | |
| $—NH_2$ | —CO—G—CO— | $—NH_2$ |

wherein:

G is an alkylene group containing 1 to 8, usually 1 to 6 carbon atoms, G' is an alkylene group containing 2 to 6, usually 2 to 4 carbon atoms, and g and g' are 1 to 6, usually 1 to 4. It is understood that the above table is merely illustrative of the more common linking groups, other linking groups being available in special situations. For example, where phenolic groups are present, such as tyrosyl groups, aryl diazonium groups may be employed. Furthermore, it is understood that the functional groups for the fluorescein derivative and ligand may be reversed, with concomitant reversal of the direction of the linking group.

The fluorescein derivatives have many desirable properties. The products have significant water solubility which allow them to be conjugated to a wide variety of polypeptides, without significantly adversely affecting the water solubility of the polypeptide, nor having the polypeptide adversely affect the spectroscopic properties of the fluorescein derivatives.

As to the spectroscopic properties of the florescein derivatives they absorb at relatively long wavelengths, generally in excess of 500 nm, more usually in excess of 510 nm. Thus, naturally occurring fluorescence which may be encountered when working with physiological fluids is substantially avoided by employing exciting light at a wavelength range which does not significantly excite the naturally occurring fluorescers. In addition, the fluorescein derivatives have relatively sharp absorption peaks and emission peaks. Because of this, efficient overlap can be obtained between fluorescers and quenchers which allow for efficient quenching up to distances of about 70Å. The fluorescein derivatives also have large Stokes shifts, so that the absorption band and emission band peaks are separated by at least 10 nm, frequently by at least 15 nm. The large Stokes shifts minimize background interference with the observed fluorescence.

The fluorescein derivatives are prepared according to conventional methods. The appropriate resorcinol and carboxylic acid or anhydride are combined in the presence of a Lewis acid e.g. zinc chloride, and the mixture heated at an elevated temperature for a sufficient time to provide the desired product. The product may then be purified by conventional means.

The conjugates can be used for determining qualitatively, semi-quantitatively or quantitatively the presence of a compound of interest in a sample. Where compounds are to be detected in physiological fluids, the fluids may include serum, urine, saliva, lymph or the like. Where the compound of interest is involved in chemical processing or ecological concerns, the sample may involve an aqueous medium, an organic medium, soil, inorganic mixtures, or the like.

As indicated previously, the fluorescein derivatives are conjugated to a compound of interest, including a receptor for an analyte or a ligand which may be measured by known immunoassay techniques. Those compounds which are analogs of the analyte, which analyte may also be referred to as a ligand, will be referred to as ligand analogs. The xanthenone conjugates of this invention are reagents which compete in an assay medium for the compound of interest or analyte in a sample. Therefore, the conjugate retains a sufficient proportion of the structure of the compound of interest to be able to compete with the compound of interest for the receptor, usually an antibody.

The analytes or their analogs, receptors or ligands, which are conjugated to the spectroscopically active fluorescein derivatives are characterized by being monoepitopic or polyepitopic.

The assays will normally involve a change of spectroscopic properties due to a change in environment about the spectroscopically active compound or the bringing together of a fluorescer-quencher pair within

sufficient proximity for the quencher to interact with the fluorescer. Alternatively, methods can be employed which involve the separation of associated and unassociated fluorescer and the detection of the fluorescer in one or both of the fractions.

In a first assay, steric exclusion is involved, in that receptors or antibodies for the ligand and for the fluorescein derivative are employed, where simultaneous binding of the receptor for the ligand and receptor for the fluorescein derivative is inhibited. Furthermore, when the receptor for the flurescein derivative (antifluorescer) is bound to the fluorescein derivative, the fluorescence of the fluorescein derivative is substantially diminished. Further reduction if not complete inhibition of fluorescence can be achieved by conjugation of quencher to the antifluorescer. This assay is extensively described in U.S. Patent No. 3,998,943 in columns 3—5.

Generally, the method involves combining the samples suspected of containing the analyte, the conjugate of the ligand and fluorescein derivative, anti-fluorescer, and the receptor for ligand or antiligand, when the ligand is the analyte. The materials are combined in an aqueous medium at a pH in the range of about 5 to 10, usually in the range of about 6 to 9, at a temperature in the range of about 10 to 45°C and the fluorescence determined either as a rate of equilibrium mode, readings being taken within about 1 second to 1 hour after all materials have been combined for a rate mode, while for an equilibrium mode, readings may be taken for as long as up to about 24 hours or longer.

In the next immunoassay technique, a fluorescer-quencher pair is employed, where one of the members of the pair is conjugated to a member of a specific binding pair, ligand and antiligand, and the other chromophor member is bound to the same or different member of the specific binding pair. For example, the fluorescer and the quencher may be bound to different molecules of antiligand, so that when the two conjugated antiligands are brought together with antigen, the fluorescer and quencher are brought within quenching distance. Alternatively, one could bind one of the chromogens to the ligand and the other chromogen to the antiligand. This assay is extensively described in U.S. Patent No. 3,996,345, column 17—23. The ratios of chromogen ligand and receptor is described in column 4—6.

The assay is carried out in substantially the same manner as described above, except that in this assay, the fluorescer conjugates and quencher conjugates are added in conjunction with the sample and the fluorescence determined in comparison to an assay medium having a known amount of the analyte.

## Experimental

The following examples are given by way of illustration and not by way of limitation.

All parts and percents not otherwise indicated are by weight, except for mixtures of liquids which are by volume. The following abbreviations are employed. TLC — thin layer chromatography; THF — tetrahydrofuran; DCC — dicyclohexyl carbodiimide; NHS — N-hydroxy succinimide; $T_3$-triiodothyronine trizma — tris(hydroxymethyl)amino-methan maleate.

### Example I

Preparation of 2,7-dimethyl-9-(3′,4′-dicarboxy-2′,5′,6′-trichlorophenyl)-6-hydroxy-3H-xanthen-3-one

A. 4-Methyl phthalic anhydride (20.0 g) was dissolved in 20% fuming sulphuric acid (25 ml) and powdered iodine (0.5 g) added. The mixture was heated to 90—100°C and chlorine gas bubbles through the solution continuously. After 24 hrs heating, 0.5 g more of iodine was added and the heating continued for 24 hrs more. After 2 days of heating, a white solid had precipitated out. The solution was cooled and diluted in 100 ml of ice-cold water and filtered to yield a white solid. The solid was washed with cold water (20 ml) and dried in vacuo. A sample of the product 3,5,6-trichloro-4-methylphthalic acid anhydride (I) was hydrolyzed to yield a white powder m.p. 226—228°C.

B. 20 g (0.075 moles) of (I) were placed in a 1-liter, 3-neck flask equipped with a mechanical stirrer and a water condenser and 400 ml 10% $K_2CO_3$ added. The slurry was heated under reflux in an oil bath at 120°C until all the solid had dissolved (about 1 hr). After adding 10 ml *tert*-butanol, 33.0 g (0.2 moles) powdered $KMnO_4$ was added in portions to the hot stirred solution. Care was taken to avoid accumulation of $KMnO_4$. An additional 100 ml of 10% $K_2CO_3$ was used to wash in the $KMnO_4$. After all $KMnO_4$ had been added, the reaction was checked by TLC. [TLC was taken by the following procedure: A sample of the reaction mixture was acidified with 6M $H_2SO_4$; the excess $KMnO_4$ was reacted with a saturated solution of oxalic acid; the clear solution was extracted with ether. The ether solution was reacted with an ether solution of diazomethane and concentrated under $N_2$. TLC was taken of the methyl ester in 100% benzene; Rf = 0.3. The Rf of the methyl ester of the diacid derived from I was 0.5.]

The reaction was stopped when all the diacid was gone by TLC. The *tert*-butanol was distilled off. The stirring slurry was acidified with 6M $H_2SO_4$ to pH 1. The excess $KMnO_4$ was removed by reaction with solid oxalic acid. Sulfuric acid (6M) was added to keep the mixture at pH 1 during the oxalic acid addition. The solution was concentrated on a rotating evaporator yielding a white slurry. Hydrochloric acid (6M) was added until the total volume has approximately 300 ml and all the solid had dissolved. The solution was stirred for 30 min at room temperature giving a fine white precipitate. The slurry was extracted with ether [3 × 400 ml]. A white inorganic salt came out in the aqueous layer. The ether was distilled off on a rotating evaporator. The resulting oil was dried by azoeotropic destillation using dry benzene. A white powder (20 g) was isolated. The powder was recrystallized in ethyl acetate-carbon tetrachloride to give 19.5 g of 3,5,6-trichlorotrimellitic acid (II). mp 238—240°C.

C. In a 250 ml flask was dissolved 10 g of the trichlorotrimellitic acid (II) in 30 ml acetic anhydride and the mixture heated at 140—145°C under $N_2$ for 45 min. After cooling, the acetic anhydride was removed on a rotating evaporator under high vacuum, while heating at 35—40°. After complete removal of acetic anhydride, the flask was left on high vacuum directly overnight to remove the last traces of acetic anhydride, and the product (III) was used directly in the next step.

D. 9,5 g of the trichlorotrimellitic anhydride (III) was mixed with powdered 4-methylresorcinol (8.5 g, dried overnight under high vacuum) in a wide mouth tube and heated in a preheated oil bath at 185—90°. Anhydrous $ZnCl_2$ (1 g) was added to the mixture and the heating continued for 1.5 hrs with occasional mixing with a spatula, a hard red mass being obtained. The contents were cooled and the red solid scraped off of the tube.

The solid was dissolved with stirring in 300 ml of 8% aq. NaOH, cooled in an ice bath and acidified with 1:1 HCl to pH 1. A yellow solid separated out. After filtering and washing with water (150 ml), the precipitate was dried under high vacuum overnight. The dry yellow solid weighed about 15.5 g.

The dried yellow powder was stirred with 200 ml of ethyl acetate overnight, the mixture filtered and the solids washed with 15 ml ethyl acetate. The ethyl acetate filtrate was concentrated on a rotating evaporator at ~35—40°C to almost dryness. The residue was stirred with 200 ml benzene for 2 hrs and filtered. The filtered solid was stirred with 200 ml of $CH_2Cl_2$ fpr 3—4 hrs and again filtered. The remaining solid weighed 9.0 g. Its TLC in THF: $CH_2Cl_2$ (1:1) indicated that it is almost pure with one major spot on TLC and a couple of minor spots moving almost with the solvent front. The yellow solid was used without further purification for the next reaction.

The above yellow solid mixture (6.0 g) was dissolved in 150 ml of freshly distilled THF (distilled over $CaH_2$) and 3.0 g DCC added. The clear solution was then stirred overnight. Next day, the white solid which had formed was filtered, the solid washed with 15 ml dry THF and the combined filtrates concentrated to dryness on a rotating evaporator at ambient temperature. To the solid was then added 200 ml n-hexane and the mixture stirred for 2 hrs to remove excess DCC. The yellow solid was filtered and washed with 50 ml n-hexane. The remaining solid is a mixture of unreacted VI and anhydride VII as shown by TLC (solvent system THF:$CH_2Cl_2$ 60:40). (VI - 2,7 - dimethyl - 9 - (2′,4′ - dicarboxy - 3′,5′,6′ - trichlorophenyl) - 6 - hydroxy - 3H - xanthen - 3 - one; VII - 2,7 - dimethyl - 9 - (3′,4′ - dicarboxy anhydride - 2′,5′,6′ - trichlorophenyl) - 6 - hydroxy - 3H - xanthen - 3 - one).

## Example II

Preparation of 2,7-dimethyl-9-(3′ or 4′-carboxamido-4′ or 3′-carboxy-2′,5′,6′-trichlorophenyl)-6-hydroxy-3H-xanthen-3-one acetic acid

The yellow solid obtained in Example I was dissolved in dry THF (300 ml) and combined with 8,5 g of 3-β-cholestanyl glycinate and the mixture stirred overnight at room temperature. The solvent was then removed and the residual solid stirred with water (150 ml) for 2 hrs. The resulting mixture was acidified with diluted HCl to pH 1 and stirring continued for 1 hr more in the cold room. The resulting yellow solid was filtered and washed with 100 ml of ice-cold water and dried *in vacuo*. Its TLC (THF:$CH_2Cl_2$ 1:1) indicated it to be a mixture of only two major compounds. The yellow solid was absorbed on silica gel (30 g) with THF and dried. The dry powder was poured over a dry column of silica gel (200 g) and eluted with a THF:$CH_2Cl_2$ mixture (1:4) with the elution followed by TLC. The faster moving spot eluant was collected and the solvent removed to give the cholestanyl ester deriative of the above indicated compound as a yellow powder (2.6 g).

Hydrolysis of the above ester was carried out by dissolving 1.5 g of the ester in 10 ml of THF, adding aqueous NaOH (1 g in 70 ml $H_2O$) and stirring the solution for 24 hrs at room temperature. A white solid separated out. The alkaline solution was extracted with ether (3 × 50 ml) and the aqueous solution acidified with conc. HCl to pH 2. A yellow solid separated out. This solution was extracted with ether (3 × 100 ml), the ether extract washed with brine solution (2 × 15 ml) and upon removal of the ether a yellow solid was obtained, which was dried *in vacuo* for 4 hrs. The residual mass was stirred with $CH_2Cl_2$ (45 ml) overnight, resulting in a yellow powder precipitating. This was filtered and washed with more $CH_2Cl_2$ (20 ml) and the final yellow solid (700 mg) dried *in vacuo*.

## Example III

Conjugation of the product of Ex. II to human IgG

a) *Preparation of the activated NHS deriative of the dye*

The product of Ex. II (60 mg) was dissolved in dry THF (1 ml) and NHS (30 mg) added, followed by the addition of DCC (30 mg) and stirring at room temperature for 3 hrs. A white solid separated out which was filtered off. The filtrate was concentrated *in vacuo* and the residue stirred for 30 min with 10 ml of n-hexane to remove excess DCC. The yellow powder was filtered off. It was used directly in the next step.

b) *Reaction of NHS ester of the fluorescein derivative with human IgG*

Human IgG (10 mg) was dissolved in 1.2 ml of 0.05 M $PO_4^{3-}$ buffer at pH 8.0 and cooled to 0—5° in an ice cold bath. A solution of 1.2 mg of the above prepared NHS ester in dry DMF (40 μl) was added during 20 min to the rapidly stirred protein solution (The pH of the solution is maintained at 8.0 during addition of NHS ester by adding a trace of solid $Na_2CO_3$). After the addition is complete, the mixture is stirred for 1.5 hrs at room temperature and then 1 ml of 2 N $NH_2OH$ (adjusted to pH 8.1) is added and the mixture stirred

for 1 hr. more in the cold room. After centrifugation of the reaction mixture, the supernatant solution was purified through a Sephadex G—25 column using 0.05 M $PO_4^{3-}$ buffer at pH 8.0. The faster moving conjugate was found to have $\lambda_{max}^{abs}$ 521—522 nm and $\lambda_{max}^{emission}$ 537—538 nm. This particular conjugate was found to have dye/protein ratio of 7.56 based on uv calculations.

Example IV

Conjugate of the product of Ex. II with triiodothyronine ($T_3$) and dextran

In general, great care was taken to avoid the exposure to light of compounds at all stages of the sequence.

A. $T_3$ (1 g) was suspended in dry methyl alcohol (15 ml) and a slow stream of hydrogen chloride gas passed through the suspension for 20 min. A clear solution was obtained. The solvent was removed on a rotating evaporator at room temperature, and the residual white solid dried in vacuo and used directly.

B. To a mixture of the above ester (70 mg) in dry DMF (1 ml) containing triethylamine (100 µl) was added the NHS ester of the product of Ex. II (prepared from 70 mg in accordance with the procedure described above) and the solution stirred overnight. The DMF was removed in vacuo and the residue treated with diluted HCl resulting in a yellow solid which was filtered, washed with water, and dried in vacuo. Its TLC (THF: $CH_2Cl_2$ 40:60) indicated that it has one major spot. The material was purified by preparative TLC using 16 plates (20 × 20 cm) and the above solvent system. The major spot was eluted with methanol and the methanol removed to yield ~40 mg product.

C. A solution of the above product (30 mg) in 1N NaOH (2 ml) was stirred at room temperature for 2 hrs. The solution was acidified with diluted HCl and the yellow solid filtered and dried. The acid was further purified by preparative TLC ($CH_2Cl_2$:MeOH:AcOH 75:25:1) and the product eluted from silica gel with methanol. After removing the methanol, the residue was dissolved in 1N NaOH, the solution filtered and acidified with diluted HCl. The resulting yellow precipitate was filtered, washed with water and dried overnight in vacuo at 65° to give the desired acid product (13 mg). Its UV spectrum in 0.05 M $PO_4^{3-}$ buffer had $\lambda_{max}^{absorption}$ 519 nm, and $\lambda_{max}^{emission}$ 533—534 nm.

D. Aminodextran was prepared from bromohydroxy propyl (BHP) activated Dextran 70 (Sigma). BHP activated Dextran 70 (500 mg) was stirred overnight with 10 ml of buffer (0.15 M $NH_4OH$ and 0.1 M $NaHCO_3$—$Na_2CO_3$, pH 9.0) at room temperature. β-Mercaptoethanol (70 µl) was added and stirring continued at room temperature for 10 hrs. After dialyzing against water at room temperature over the weekend, the total volume after dialysis was about 14 ml (contains about 500 mg of Dextran 70).

E. A solution of 10 mg of the acid obtained in step C in dry THF (1 ml), containing DCC (9 mg) and NHS (5 mg), was stirred overnight. A white solid separated out. After filtering, the filtrate was concentrated in vacuo. The residue was triturated with hexane (10 ml) and the yellow solid (~20 mg) separated. On TLC examination in THF:$CH_2Cl_2$ (1:1), the yellow solid appeared to be mostly the NHS ester (higher Rf) with a small amount of starting material (lower Rf).

F. The aminodextran solution (300 µl) prepared above was diluted with 300 µl of 0.1 N $NaHCO_3$—$Na_2CO_3$ solution (pH 9.0), and to this was added a solution of 0.5 mg of the NHS ester prepared in step E in 25 µl of THF. The solution was stirred at room temperature for 1.5 hrs. To this solution was then added 0.3 ml of 3N $NH_2OH$ solution (pH 8.0) and the mixture stirred for 45 min at room temperature. After centrifugation for 2 min, the supernatant was purified over a G—25 Sephadex column (1 × 30 cm) using 0.1 N $NaHCO_3$—$Na_2CO_3$ buffer (pH 9.0). The faster moving conjugate was collected (total volume of conjugate after chromatographing is about 2 ml). It had $\lambda_{max}^{absorption}$ 521—522 nm, $\lambda_{max}^{emission}$ 537—538 nm. Its quantum yield was 43% as compared to the parent fluorescein derivative of Ex. II (based on emission peak area when both are excited at 500 nm).

Example V

Preparation of 2,7-dimethyl-9-(2′,4′ or 5′ dicarboxyphenyl)-6-hydroxy-3H-xanthen-3-one and the 4,5-dichloro derivative.

A. In a reaction flask was combined 1.35 g 4-methylresorcinol, 1 g trimellitic anhydride and 100 mg $ZnCl_2$ and the mixture heated at 195—200°C for 15 min. The resulting solid was triturated with water and filtered. The precipitate was dissolved in 5% NaOH, stirred for 5 min, filtered and the filtrate acidified with diluted HCl to pH 2. The resulting solid was recrystallized from methanol to yield an orange red solid. mp >280°C.

B. A portion of the above product was dissolved in DMF, and 2.2 equiv. of chlorine in glacial acetic acid added. When no further reaction appeared to be occurring, the product was isolated and purified by preparative TLC using $CHCl_3$: MeOH (80:20).

Example VI

Preparation of 2,7-di(2″-carboxyethyl)-9-(2′-carboxyphenyl)-6-hydroxy-3H-xanthen-3-one

Into a reaction flask was introduced 1.1 g of 4-(2′-carboxyethyl)resorcinol, 0.45 g phthalic anhydride and 250 mg $ZnCl_2$ and the mixture heated at 160—170°C for 0.5 hr. After treating with water and filtering, the solid was dissolved in 5% NaOH, the solution stirred for 15 min, the filtrate acidified with diluted HCl to pH 2 and the resulting yellow solid filtered and dried. The product was triturated with ethyl acetate and further purified by preparative TLC ($CHCl_3$:MeOH:$CH_3COOH$ glac. 80:20:0.5).

Example VII

Preparation of 2,7-di(3''-carboxypropyl)-9-(2',4'- and 3',4'-dicarboxy-3',5',6' and 2',5',6'-trichlorophenyl)-6-hydroxy-3H-xanthen-3-one.

Following the prior procedures, into a reaction flask was introduced 2.6 g 4-(3'-carboxypropyl)-resorcinol, 1.95 g 3,5,6-trichloro-1,2,4-benzenetricarboxylic acid and 100 mg $ZnCl_2$ and the mixture heated at 180—185°C for 40 min. The mixture was worked up as previously described and the product purified by preparative TLC using $CHCl_3$:MeOH:HOAc 80:20:1.

Table III reports the spectroscopic properties of the described compounds.

TABLE III

| Ex. | $\lambda_{max}^{absorption\,1}$ | $\lambda_{max}^{emission\,1}$ | $\varepsilon \times 10^{3\,1}$ | $\phi^2$, % |
|---|---|---|---|---|
| I | 516 | 531 | 70.5 | 76 |
| II | 518 | 532—3 | 72.0 | 70 |
| V A | 500 | 518—9 | 67.3 | 92 |
| B | 511—2 | 523—5 | | 100 |
| VI | 500—1 | 521—2 | 70.0 | 88 |
| VII | 519 | 535 | 69.0 | 64 |

1. In 0.05 M $PO_4^{3-}$ buffer, pH 8.0
2. Compared to fluorescein on the basis of emission peak measured in 0.05 M $PO_4^{3-}$ excited at 475 nm.

It is evident from the previous examples that the fluorescein derivatives have many desirable properties. The compounds absorb at wavelengths at or in excess of 500, having large Stokes shifts, and their spectroscopic properties are not adversely affected by being conjugated to proteins.

In order to demonstrate further the utility of the subject inventions, immunoassays were carried out, where the fluorescent label compound was a fluorescein derivative as defined above. The assay was concerned with immunoglobulin G. The following solutions were employed in preparing reagents for carrying out the assay:

Buffer 0.01 M $Na_2HPO_4$, 0.15 M NaCl, 2% PEG 6000, 0.05% $NaN_3$, pH 8.0.

Reagent B diluent: 0.05 M trizma base, 0 02 M glycine, 0.01 M benzamidine-HCl, 0.15 M NaCl, 0.05% $NaN_3$, pH 8.0.

Reagent A diluent: 0.05 M trizma base, 1- cholate, 0.01 M benzamidine-HCl, 0.05% $NaN_3$, pH 8.0.

Calibrator diluent: 0.01 M $Na_2HPO_4$, 0.15 M NaCl, 0.01 M benzamidine-HCl, 0.01% $NaN_3$, pH 7.2.

Reagent A is a rhodamine-anti(hIgG) conjugate diluted 1:30 with reagent A diluent and having a rhodamine/protein ratio of 11. Reagent B is the compound of Example II conjugated to hIgG diluted 1:26.7 with reagent B diluent and having a fluorescer/protein ratio of about 2.

The calibrators are derived from Freon$^R$-dextran sulfate treated serum, diluted with calibrator diluent, except for the 24 mg/ml calibrator which employs undiluted serum. The negative calibrator is assay buffer.

The assay is performed with a Varian Fluorichrom$^R$/fluorometer (modified), at 25°C employing as the gain a fluorescence signal set to 1600 using a standard solution of $3.8 \times 10^{-9}$ M of the fluorescer itself.

The assay is performed by combining 16 µl of the calibrator or sample, diluted with 400 µl of assay buffer following by the addition of 50 µl each of reagent A and reagent B and an additional 400 µl of assay buffer. After 5 sec, the change in fluorescence is read over a 6 sec period, to determine the rate of change in fluorescence.

A standard curve was prepared having the following hIgG mg/ml concentrations: 0, 1.6, 5.6, 11.2, 17.6 and 24.0.

A number of samples were then employed and the hIgG determined and compared with commercially available RID or nephelometric techniques. The following table indicates the results.

TABLE III

| Comparative Method | No. of Samples | Intercept | Slope | Correlation Coefficient | Standard Error |
|---|---|---|---|---|---|
| RID | 50 | −0.12 | 1.12 | 0.97 | 1.49 |
| Nephelometry (1) | 25 | 3.87 | 0.72 | 0.91 | 1.76 |
| Nephelometry (2) | 12 | 3.25 | 0.59 | 0.96 | 1.65 |

It is evident from the above results, that there is a consistent relationship between the subject method employing the xanthenone conjugates of the invention and other commercially available assays. There is an evident bias in the nephelometric results, possibly due to each of the techniques employing its own method of valuation.

The present invention provides novel xanthenone conjugates which have important spectroscopic properties, providing for absorption at long wavelengths, high extinction coefficients, sharp absorption bands and fluorescent bands and large Stokes shifts, i.e. substantial spacing between absorption and fluorescence bands. These properties are particularly desirable and important to the development of fluorescent techniques for the detection of a wide variety of materials.

**Claims**

1. Conjugates of 2,7-dialkylsubstituted-9-substituted xanthen-6-hydroxy-3H-3-ones covalently linked at the 2 or 9 position to a heteroatom of an organic compound having a molecular weight of at least 125, said xanthenone having an absorption of at least about 500 nm, a Stokes shift of at least 10 nm and an extinction coefficient of at least about 65000.

2. Conjugates according to claim 1, wherein the xanthenone has at least two chloro substituents at other than the 1,8 positions.

3. Conjugates according to claim 1, wherein the xanthenone conjugate has the general formula:

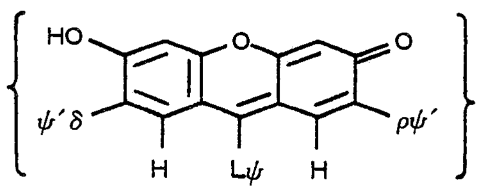
$(\alpha)$
$\nu$

wherein:

α is an organic compound covalently bonded to one of ψ or ψ';

ρ is an aliphatic hydrocarbon group containing 1 to 12 carbon atoms.

ψ' is a hydrogen atom or a non-oxo-carbonyl group;

L is a bond or divalent group;

ψ is a linking group bonded to a α or a group terminating in a heteroatom containing a functional group capable of linking when not bonded to α; and

υ is 1 to the molecular weight of α divided by 500.

4. Conjugates according to claim 1, wherein the xanthenone conjugate has the general formula:

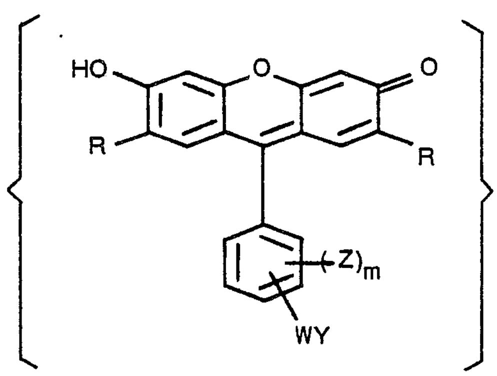
A
n

wherein:

R is an alkyl carboxyalkyl group containing 1 to 4 carbon atoms;

Z is a carboxy group;

A is a ligand or receptor;

W is a bond or divalent group containing 0 to 8 carbon atoms;

Y is a methylene group or a heteroatom containing a linking group;

m is 0 to 3;

n is 1 to the molecular weight of A divided by 500; and

said portion in the brackets of said formula having from 0 to 6 chloro substituents at other than the 1,8-position of the xanthene portion of the molecule.

5. Conjugates according to claim 1, wherein the xanthenone conjugate has the general formula:

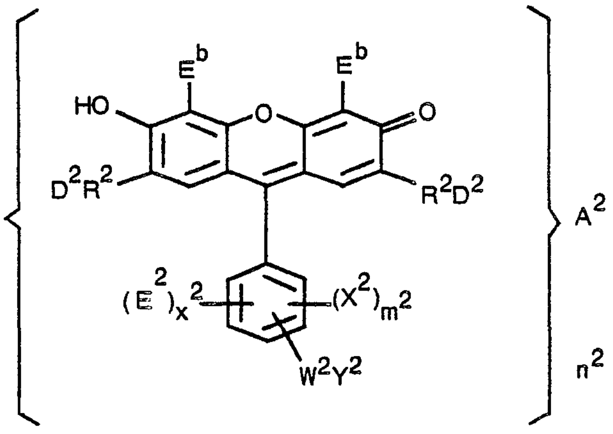

wherein:

$E^b$ is a hydrogen or chlorine atom;

$E^2$ is a chlorine atom;

$Z^2$ is a carboxy group;

$R^2$ is an alkylene group containing 1 to 6 carbon atoms;

$D^2$ is a hydrogen atom or a carboxy group;

$W^2$ is a bond, an alkylene, carboxamidoalkylene, or poly(carboxamidoalkylene) group of the formula $(-CONH-(CH_2)_{1-2}-)_a$ wherein a is 1 to 4;

$Y^2$ is non-oxo-carbonyl, carbamoyl, thiocarbamoyl, amino, methylene, sulfonyl, oxy or thio group;

$A^2$ is a poly(amino acid) ligand or a receptor;

$m^2$ is 0 to 3;

$x^2$ is 0 to 4; and

$n^2$ is 1 to the molecular weight of $A^2$ divided by 500.

6. Conjugates according to claim 5, wherein $A^2$ is a poly(amino acid) having a molecular weight of from about 2,000 to 600,000.

7. Conjugates according to claim 1, wherein the xanthenone conjugate has the general formula:

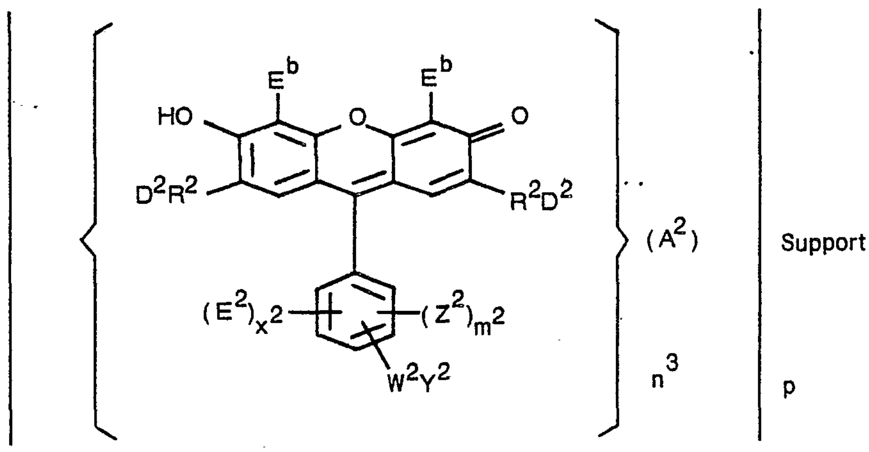

wherein:

$n^3$ is 1 to the molecular weight of $A^2$ divided by 500, with the proviso that when q is 0, $n^3$ is 1;

p is at least 1 and up to the molecular weight of Support divided by 500;

Support is a molecule having a molecular weight of at least about 10,000 and having a plurality of functional groups for linking;

the group in the brackes is joined by a bond or linking group to the Support;

$E^b$ is a hydrogen or chlorine atom;

$E^2$ is a chlorine atom;

$Z^2$ is a carboxy group;

$R^2$ is an alkylene group containing 1 to 3 carbon atoms;

$D^2$ is a hydrogen atom or a carboxy group;

$W^2$ is a bond, an alkylene, carboxamidoalkylene, or poly(carboxyamidoalkylene) group of the formula $(-CONH-(CH_2)_{1-2}-)_a$ wherein a is 1 to 4;

$Y^2$ is non-oxo-carbonyl, carbamoyl, thiocarbamoyl, amino, methylene, sulfonyl, oxy or thio group;

$A^2$ is a poly(amino acid) ligand or receptor bonded to $Y^2$;

$m^2$ is 0 to 3;

$x^2$ is 0 to 4.

8. Conjugates according to claim 7, wherein said support is a polysaccharide.

9. Conjugates according to any of claims 7 and 8, wherein $A^2$ is a hapten having a molecular weight of up to about 2,000 and $Y^2$ is a non-oxo-carboxyl group.

10. Conjugates according to any of claims 7 and 8, wherein $A^2$ is a poly(amino acid) group having a molecular weight of from about 2,000 to 600,000 and $Y^2$ is a non-oxo-carbonyl group.

11. A method for performing a fluorescence immunoassay, characterized in that a conjugate according to any of claims 1 to 10 is used.

**Patentansprüche**

1. Konjugate von in der 2- und 7-Stellung dialkylsubstituierten und in der 9-Stellung substituierten Xanthen-6-hydroxyl-3H-3-onen, die in der 2- oder 9-Stellung mit einem Heteroatom einer organischen Verbindung mit einem Molekulargewicht von mindestens 125 verknüpft sind, wobei das genannte Xanthenon eine Absorption bei mindestens etwa 500 nm, eine Stokes-Verschiebung von mindestens 10 nm und einen Extinktionskoeffizienten von mindestens etwa 65 000 aufweist.

2. Konjugate gemäß Anspruch 1, in denen das Xanthenon mindestens zwei Chlorsubstituenten in anderen als den 1,8-Stellungen aufweist.

3. Konjugate gemäß Anspruch 1, in denen das Xanthenon Konjugat die folgende allgemeine Formel aufweist:

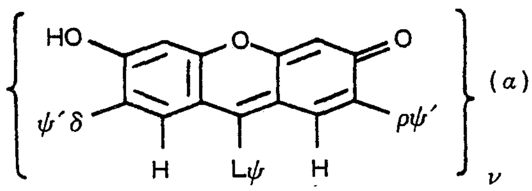

in der:

α eine organische Verbindung darstellt, die kovalent an ψ oder ψ′ gebunden ist;

ρ einen aliphatischen Kohlenwasserstoffrest mit 1 bis 12 Kohlenstoffatomen bedeutet,

ψ′ ein Wasserstoffatom oder eine Nicht-oxocarbonylgruppe bedeutet,

L eine Bindung oder einen zweiwertigen Rest darstellt,

ψ einen an α gebundenen Brückenrest, oder einen Rest mit einem Heteroatom am Ende, der eine funktionelle Gruppe enthält, die als Verknüpfung dienen kann, wenn sie nicht an α gebunden ist, bedeutet; und

ν einen Wert von 1 bis zum Molekulargewicht von α, dividiert durch 500 hat.

4. Konjugate gemäß Anspruch 1, in denen das Xanthenon-Konjugat die folgende allgemeine Formel aufweist:

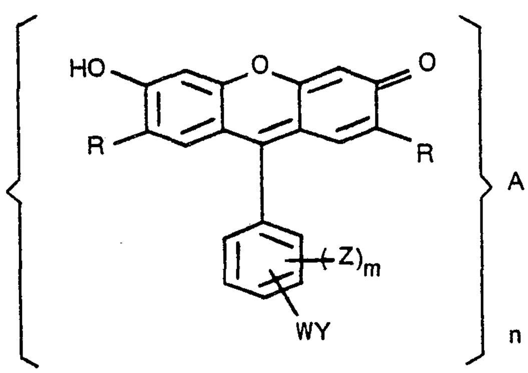

in der:

R einen Alkyl- oder Carboxyalkylrest mit 1 bis 4 Kohlenstoffatomen bedeutet,

Z eine Carboxylgruppe ist,

A einen Ligand oder Rezeptor bedeutet,

W eine Bindung oder einen zweiwertigen Rest mit 0 bis 8 Kohlenstoffatomen bedeutet,

Y eine Methylengruppe oder ein Heteroatom als Brückenrest darstellt,

m den Wert 0 bis 3 aufweist,

n einen Wert von 1 bis zum Molekulargewicht von A dividiert durch 500 hat, und

der Rest in den Klammern der obigen Formel 0 bis 6 Chlorsubstituenten an anderen als den 1,8-Stellungen der Xanthenhälfte des Moleküls bedeutet.

5. Konjugate gemäß Anspruch 1, in denen das Xanthenon-Konjugat die folgende allgemeine Formel aufweist:

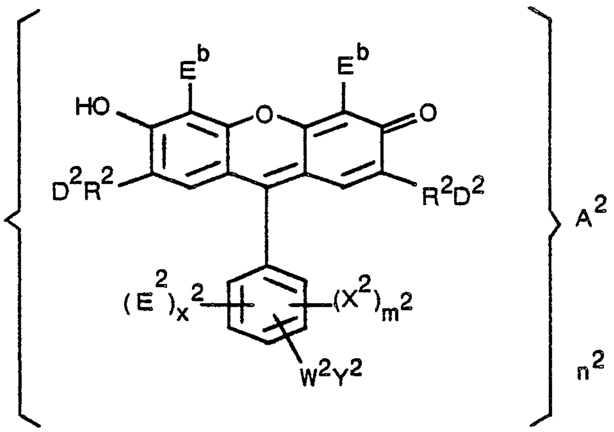

in der:

$E^b$ ein Wasserstoff- oder Chloratom ist,

$E^2$ ein Chloratom bedeutet,

$Z^2$ eine Carboxylgruppe darstellt,

$R^2$ einen Alkylenrest mit 1 bis 6 Kohlenstoffatomen bedeutet,

$D^2$ ein Wasserstoffatom oder eine Carboxylgruppe bedeutet,

$W^2$ eine Bindung, einen Alkylenrest, einen Carboxamidoalkylenrest oder einen Polycarboxamidoalkylenrest der allgemeinen Formel $(—CONH—(CH_2)_{1-2}—)_a$, in der a den Wert 1 bis 4 hat, darstellt,

$Y^2$ eine Nicht-oxocarbonyl-, Carbamoyl-, Thiocarbamoyl-, Amino-, Methylen-, Sulfonyl-, Oxy- oder Thiogruppe bedeutet.

$A^2$ einen Polyaminosäure-Liganden oder einen Rezeptor darstellt,

$m^2$ den Wert 0 bis 3 hat,

$x^2$ den Wert 0 bis 4 hat, und

$n^2$ einen Wert von 1 bis zum Molekulargewicht von $A^2$, dividiert durch 500 aufweist.

6. Konjugate gemäß Anspruch 5, in denen $A^2$ eine Polyaminosäure mit einem Molekulargewicht von etwa 2000 bis 600 000 bedeutet.

7. Konjugate gemäß Anspruch 1, in denen das Xanthenon-Kojugat die folgende allgemeine Formel aufweist:

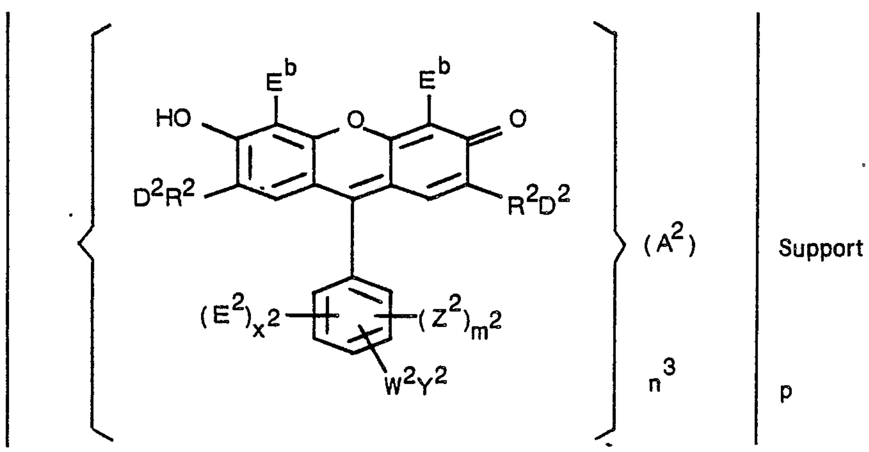

in der:

$n^3$ einen Wert von 1 bis zum Molekulargewicht von $A^2$, dividiert durch 500 bedeutet, mit der Maßgabe, daß, wenn q den Wert 0 hat, $n^3$ den Wert 1 hat,

p einen Wert von mindestens 1 bis zum Molekulargewicht des Trägers, dividiert durch 500 aufweist,

der Träger ein Molekül mit einem Molekulargewicht von mindestens etwa 10 000 ist mit einer Vielzahl von funktionellen Gruppen zur Verknüpfung,

der Rest in den Klammern durch eine Bindung oder einen Brückenrest an den Träger gebunden ist,

$E^b$ ein Wasserstoff- oder Chloratom bedeutet,

$E^2$ ein Chloratom ist,

$Z^2$ eine Carboxylgruppe darstellt,

$R^2$ einen Alkylenrest mit 1 bis 3 Kohlenstoffatomen bedeutet,

$D^2$ ein Wasserstoffatom oder eine Carboxylgruppe darstellt,

$W^2$ eine Bindung, einen Alkylenrest, einen Carboxamidoalkylenrest oder einen Polycarboxyamidoalkylenrest der allgemeinen Formel $(—CONH—(CH_2)_{1-2}—)_a$, in der a den Wert 1 bis 4 hat, bedeutet,

$Y^2$ eine Nicht-oxocarbonyl-, Carbamoyl-, Thiocarbamoyl-, Amino-, Methylen-, Sulfonyl-, Oxy- oder Thiogruppe bedeutet.

$A^2$ einen Polyaminosäure-Liganden oder Rezeptor bedeutet,
$m^2$ den Wert 0 bis 3 hat, und
$x^2$ den Wert 0 bis 4 hat.

8. Konjugate gemäß Anspruch 7, in denen der genannte Träger ein Polysaccharid ist.

9. Konjugate gemäß jedem der Ansprüche 7 oder 8, in denen $A^2$ ein Hapten mit einem Molekulargewicht von bis zu etwa 2000 und $Y^2$ eine Nicht-oxocarbonylgruppe bedeutet.

10. Konjugate gemäß jedem der Ansprüche 7 oder 8, in denen $A^2$ einen Polyaminosäurerest mit einem Molekulargewicht von etwa 2000 bis 600 000 und $Y^2$ eine Nicht-oxocarbonylgruppe bedeutet.

11. Verfahren zur Durchführung eines Fluoreszenz-Immuntests, dadurch gekennzeichnet, daß ein Konjugat gemäß jedem der Ansprüche 1 bis 10 verwendet wird.

**Revendications**

1. Conjugués de 2,7-dialkyl-9-xanthène substitué-6-hydroxy-3H-3-ones en liaison de covalence en position 2 ou en position 9 avec un hétéroatome d'un composé organique ayant un poids moléculaire d'au moins 125, ladite xanthénone ayant une absorption d'au moins environ 500 nm, une dérive de Stokes d'au moins 10 nm et un coefficient d'extinction d'au moins environ 65 000.

2. Conjugués suivant la revendication 1, dans lesquels la xanthénone porte au moins deux substituants chloro dans des positions autres que les positions 1,8.

3. Conjugués suivant la revendication 1, le conjugué de xanthénone répondant à la formule générale:

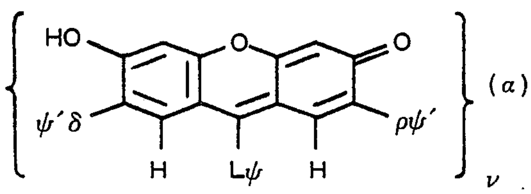

dans laquelle:

α est un composé organique en liaison de covalence avec l'un ou l'autre de ψ ou ψ';
ρ est un groupe hydrocarboné aliphatique contenant 1 à 12 atomes de carbone.
ψ' est un atome d'hydrogène ou un groupe carbonyle non oxo;
L est une liaison ou un groupe divalent;
ψ est un groupe de jonction lié à α ou un groupe se terminant par un hétéroatome, contenant un grupe fonctionnel capable de jonction lorsqu'il n'est pas lié à α; et
ν a une valeur allant de 1 au poids moleculaire de α divisé par 500.

4. Conjugués suivant la revendication 1, le conjugué de xanthénone répondant à la formule générale:

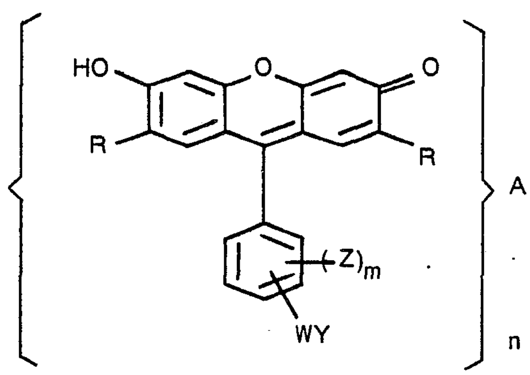

dans laquelle:

R est un groupe alkyle ou carboxyalkyle contenant 1 à 4 atomes de carbone;
Z est un groupe carboxy;
A est un ligand ou un récepteur;
W est une liaison ou un groupe divalent contenant 0 à 8 atomes de carbone;
Y est un groupe méthylène ou un groupe de jonction contenant un hétéroatome;
m a une valeur de 0 à 3;
n a une valeur de 1 au poids moléculaire de A divisé par 500; et
ladite portion entre les accolades de ladite formule ayant 0 à 6 substituants chloro dans des positions autres que les positions 1 et 8 de la portion de xanthène de la molécule.

5. Conjugués suivant la revendication 1, le conjugué de xanthénone répondant à la formule générale:

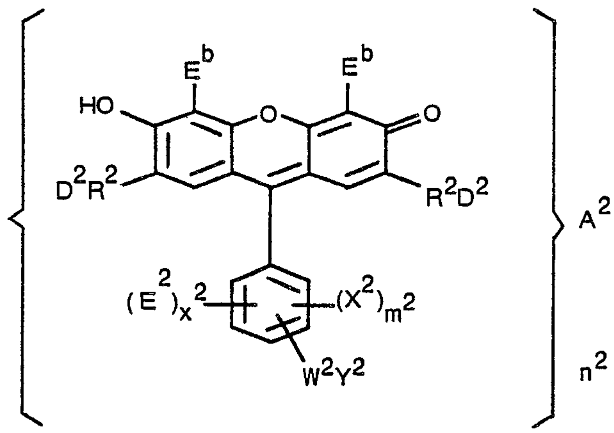

dans laquelle:

$E^b$ est un atome d'hydrogène ou de chlore;

$E^2$ est un atome de chlore;

$Z^2$ est un groupe carboxy;

$R^2$ est un groupe alkylène contenant 1 à 6 atomes de carbone;

$D^2$ est un atome d'hydrogène ou un groupe carboxy;

$W^2$ est une liaison, un groupe alkylène, carboxamidoalkylène ou poly(carboxamidoalkylène) de formule $(-CONH-(CH_2)_{1-2}-)_a$ dans laquelle *a* a une valeur de 1 à 4;

$Y^2$ est un groupe carbonyle non oxo, un groupe carbamoyle, thiocarbamoyle, amino, méthylène, sulfonyle, oxy ou thio;

$A^2$ est un ligand poly(amino-acide) ou un récepteur;

$m^2$ a une valeur de 0 à 3;

$x^2$ a une valeur de 0 à 4; et

$n^2$ a une valeur allant de 1 au poids moléculaire de $A^2$ divisé par 500.

6. Conjugués suivant la revendication 5, dans lesquels $A^2$ est un poly(amino-acide) ayant un poids moléculaire d'environ 2000 à 600 000.

7. Conjugués suivant la revendication 1, le conjugué de xanthénone répondant à la formule générale:

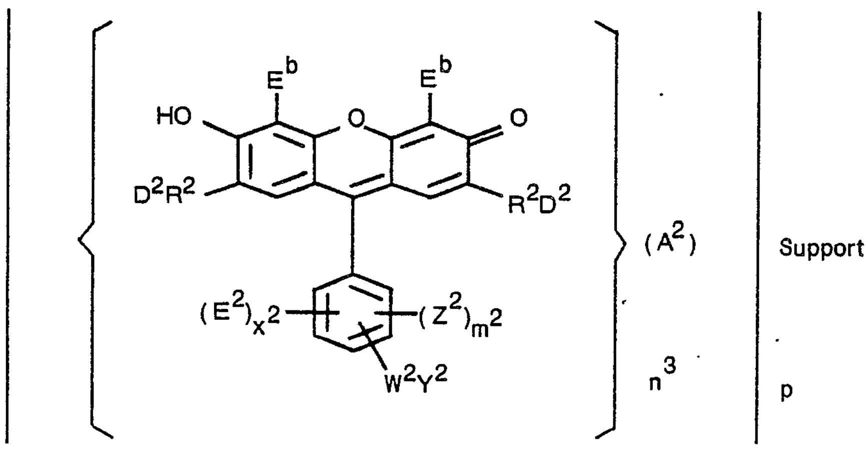

dans laquelle:

$n^3$ a une valeur allant de 1 au poids moléculaire de $A^2$ divisé par 500, sous réserve que lorsque q est égal à 0, $n^3$ soit égal à 1;

p a une valeur allant d'au moins 1 au poids moléculaire du Support divisé par 500;

le Support est une molécule ayant un poids moléculaire d'au moins environ 10 000 et portant plusieurs groupes fonctionnels de jonction;

le groupe entre les accolades est relié au Support par une liaison ou par un groupe de jonction;

$E^b$ est un atome d'hydrogène ou de chlore;

$E^2$ est un atome de chlore;

$Z^2$ est un groupe carboxy;

$R^2$ est un groupe alkylène contenant 1 à 3 atomes de carbone;

$D^2$ est un atome d'hydrogéne ou un groupe carboxy;

$W^2$ est une liaison, un groupe alkylène, carboxamidoalkylène ou poly(carboxamidoalkylène) de formule $(-CONH-(CH_2)_{1-2}-)_a$ dans laquelle *a* a une valeur de 1 à 4;

$Y^2$ est un groupe carbonyle non oxo, carbamoyle, thiocarbamoyle, amino, méthylène, sulfonyle, oxy ou thio;

$A^2$ est un ligand poly(amino-acide) ou un récepteur lié à $Y^2$;

$m^2$ a une valeur de 0 à 3;

$x^2$ a une valeur de 0 à 4.

8. Conjugués suivant la revendication 7, dans lesquels le support est un polysaccharide.

9. Conjugués suivant l'une quelconque des revendications 7 et 8, dans lesquels $A^2$ est un haptène ayant un poids moléculaire allant jusqu'à environ 2000 et $Y^2$ est un groupe carbonyle non oxo.

10. Conjugués suivant l'une quelconque des revendications 7 et 8, dans lesquels $A^2$ est un groupe poly(amino-acide) ayant un poids moléculaire d'environ 2000 à 600 000 et $Y^2$ est un groupe carbonyle non oxo.

11. méthode pour exécuter un immuno-essai par fluorescence, caractérisée en ce qu'elle utilize un conjugué suivant l'une quelconque des revendications 1 à 10.